# EUROPEAN PATENT APPLICATION

(11) **EP 4 847 413 A1**
(43) Date of publication of application: **02.12.2026**
(21) Application number: 25744590.8
(22) Date of filing: 21.01.2025
(51) Int. Cl.: B65D 75/64, B65D 25/02, A61B 5/15, A61B 5/00

(54) **SEALED PACKAGING BOX AND ANALYTE MONITORING SYSTEM COMPRISING SAME**

(30) Priority: 25.01.2024 CN 202420185718 U
(71) Applicant: SHENZHEN SISENSING CO., LTD., Shenzhen Guangdong 518110 (CN)
(72) Inventor: LI, Yunfeng, Shenzhen, Guangdong 518110 (CN)
(74) Representative: Vossius & Partner Patentanwälte Rechtsanwälte mbB
(86) International application number: PCT/CN2025/073767
(87) International publication number: WO 2025/157151

(57) **Abstract**

The present disclosure describes a sealed packaging box(300) and an analyte monitoring system(10) including the sealed packaging box(300). The sealed packaging box(300) includes a main body portion(310), a sealing portion(320), and a holding portion(330), wherein the main body portion(310) has an opening portion(311) and a chamber(312) communicated with the outside via the opening portion(311), the sealing portion(320) is adhered to the opening portion(311) and seals the chamber(312), and the holding portion(330) is at least partially attached to a peripheral edge of the sealing portion(320) to separate the sealing portion(320) from the opening portion(311) by utilizing the holding portion(330). According to the present disclosure, the ease of use of the sealed packaging box(300) can be improved.

## Description

### BACKGROUND OF INVENTION

### Field Of Invention

The present disclosure generally relates to the field of biomedical engineering, and more particularly relates to a sealed packaging box and an analyte monitoring system including the sealed packaging box.

### Description Of Related Art

A packaging box is used for storing various products, and is an indispensable part of the modern society. In an analyte monitoring system, in order to meet medical and health needs, it is necessary to use the packaging box for sealing a medical instrument. However, the use of the packaging box also causes some problems, such as how a sealing film of the packaging box is separated from a box body. In order to meet the need of a user, the design of facilitating the tearing of the sealing film of the packaging box has emerged.

The design of a conventional sealing film is mainly the design of adopting easy-tear sealing. The easy-tear sealing design is convenient for the user to pinch a protruding sealing film part to achieve an easy-tear function in a manner of providing the protruding sealing film part.

However, generally the protruding sealing film part is relatively thin and smooth, which is likely to cause the sealing film to slip off a hand of the user, and when the user pinches the sealing film and lifts the sealing film upwards to open with the hand, it is likely to cause slip of the hand, so that the sealing film often cannot be torn successfully.

### SUMMARY OF INVENTION

The present disclosure is provided in view of the above conditions in the prior art, and an object thereof is to provide a sealed packaging box and an analyte monitoring system including the sealed packaging box, which can improve the ease of use of the sealed packaging box.

To this end, a first aspect of the present disclosure provides a sealed packaging box, including a main body portion, a sealing portion, and a holding portion, wherein the main body portion has an opening portion and a chamber communicated with the outside via the opening portion, the sealing portion is adhered to the opening portion and seals the chamber, and the holding portion is at least partially attached to a peripheral edge of the sealing portion to separate the sealing portion from the opening portion by utilizing the holding portion.

In the sealed packaging box involved in the first aspect of the present disclosure, the holding portion is at least partially attached to the peripheral edge of the sealing portion. In this case, the holding portion and the sealing portion may be pinched simultaneously, so that the possibility that the sealing portion slips off the hand of the user may be reduced, thereby being convenient for the user to utilize the holding portion attached to the sealing portion for driving the sealing portion to move, so as to separate the sealing portion from the opening portion, and then the ease of use of the sealed packaging box may be improved.

In addition, in the sealed packaging box involved in the first aspect of the present disclosure, optionally, the sealing portion has a covering portion corresponding to the opening portion and a tearing portion extending outwards from the covering portion and beyond a peripheral edge of the opening portion, and the holding portion is at least partially attached to the tearing portion. In this case, the tearing portion extends outwards from the covering portion and beyond the peripheral edge of the opening portion, so that a degree of coverage of the opening portion by the sealing portion may be increased, and thus the sealability of the sealed packaging box may be improved.

In addition, in the sealed packaging box involved in the first aspect of the present disclosure, optionally, the holding portion is formed integrally with the opening portion, and a breaking stress between the holding portion and the opening portion is smaller than a breaking stress of the opening portion or a breaking stress of the holding portion. In this case, the holding portion is formed integrally with the opening portion, so that machining may be facilitated, and the sealability of the sealed packaging box may be improved. In addition, the breaking stress between the holding portion and the opening portion is smaller than the breaking stress of the opening portion or the breaking stress of the holding portion, thereby being conducive to breaking the connection of the holding portion and the opening portion by the user, and thus being able to be convenient for the user to subsequently pinch the holding portion attached to the sealing portion and move the holding portion towards a direction away from the opening portion to separate the sealing portion from the opening portion.

In addition, in the sealed packaging box involved in the first aspect of the present disclosure, optionally, the holding portion has a connecting portion connected to the opening portion, and a thickness of the connecting portion is less than a thickness of the opening portion. In this case, the thickness of the connecting portion is less than the thickness of the opening portion, so that the breaking stress between the holding portion and the opening portion may be reduced, thereby being conducive to breaking the holding portion and the opening portion from a position of the connecting portion by the user, and thus being able to be convenient for the user to subsequently utilize the holding portion for separating the sealing portion from the opening portion.

In addition, in the sealed packaging box involved in the first aspect of the present disclosure, optionally, anti-slip texture is provided on a surface of the holding portion facing away from the sealing portion. In this case, a friction coefficient of the holding portion may be increased, and the possibility that the holding portion slips off the hand of the user may be reduced. In addition, the anti-slip texture is provided on the surface of the holding portion facing away from the sealing portion, so that the tightness of adhesion between the sealing portion and the holding portion may be improved, and the sealability of the sealed packaging box may be improved as compared to the manner in which the anti-slip texture is provided on a surface of the holding portion attached to the sealing portion.

In addition, in the sealed packaging box involved in the first aspect of the present disclosure, optionally, a hardness of the holding portion is not less than a hardness of the sealing portion and/or a thickness of the holding portion is not less than a thickness of the sealing portion. In this case, the hardness of the holding portion is not less than the hardness of the sealing portion or the thickness of the holding portion is not less than the thickness of the sealing portion, so that the holding portion may be easily pinched, thereby being convenient for the user to subsequently utilize the holding portion attached to the sealing portion for separating the sealing portion from the opening portion, and thus being able to improve the ease of use of the sealed packaging box. In addition, the hardness of the holding portion is not less than the hardness of the sealing portion, and the thickness of the holding portion is not less than the thickness of the sealing portion, so that the holding portion may be more easily pinched.

In addition, in the sealed packaging box involved in the first aspect of the present disclosure, optionally, the sealing portion is hot-pressed to the opening portion and the holding portion. In this case, the tightness of adhesion between the sealing portion and the opening portion and between the sealing portion and the holding portion may be improved, and the sealability of the sealed packaging box may be improved.

In addition, in the sealed packaging box involved in the first aspect of the present disclosure, optionally, the main body portion has a protrusion formed by protruding from the opening portion towards the holding portion, and a peripheral curvature of the protrusion is greater than a peripheral curvature of the opening portion. In this case, the main body portion has the protrusion, and as compared to a case where there is no protrusion, when starting to separate the sealing portion from the opening portion, areas of the sealing portion and the opening portion needing to be separated may be reduced, thereby being convenient for the user to separate the sealing portion from the opening portion with a smaller force.

In addition, in the sealed packaging box involved in the first aspect of the present disclosure, optionally, the sealing portion has a covering portion corresponding to the opening portion and a tearing portion extending outwards from the covering portion and beyond an edge of the opening portion, the covering portion is formed integrally with the tearing portion, the covering portion is hot-pressed to the opening portion, the tearing portion is hot-pressed to the holding portion, the holding portion is formed integrally with the opening portion, the main body portion has a protrusion formed by protruding from the opening portion towards the holding portion, a peripheral curvature of the protrusion is greater than a peripheral curvature of the opening portion, the holding portion has a connecting point connected to the protrusion, a thickness of the connecting point is less than a thickness of the opening portion, a hardness of the holding portion is greater than a hardness of the sealing portion, and anti-slip texture is provided on a surface of the holding portion facing away from the sealing portion. In this case, the covering portion is formed integrally with the tearing portion, and the holding portion is formed integrally with the opening portion, so that machining may be facilitated, and the sealability of the sealed packaging box may be improved. In addition, the covering portion is hot-pressed to the opening portion, and the tearing portion is hot-pressed to the holding portion, so that the tightness of adhesion between the sealing portion and the opening portion and between the sealing portion and the holding portion may be improved, and the sealability of the sealed packaging box may be improved. In addition, the main body portion has the protrusion, and as compared to a case where there is no protrusion, when starting to separate the sealing portion from the opening portion, areas of the sealing portion and the opening portion needing to be separated may be reduced, thereby being convenient for the user to separate the sealing portion from the opening portion with a smaller force. In addition, the holding portion has the connecting point connected to the protrusion, and the connecting point is a connecting portion, so that a breaking stress of the connecting portion may be decreased by reducing a volume of the connecting portion, and the holding portion may be easily broken from the connecting portion. In addition, the thickness of the connecting point is less than the thickness of the opening portion, thereby reducing the breaking stress between the holding portion and the opening portion, and being conducive to breaking the holding portion and the opening portion from a position of the connecting portion by the user. In addition, the anti-slip texture is provided on the surface of the holding portion facing away from the sealing portion, so that the friction coefficient of the holding portion may be increased, the possibility that the holding portion slips off the hand of the user may be reduced. As compared to the manner in which the anti-slip texture is provided on the surface of the holding portion attached to the sealing portion, the tightness of adhesion between the sealing portion and the holding portion may also be improved, and thus the sealability of the sealed packaging box may be improved.

A second aspect of the present disclosure provides an analyte monitoring system, including the sealed packaging box involved in the first aspect of the present disclosure, a sensor assembly, an electronic assembly, and an exertion device, wherein the sealed packaging box is configured to accommodate and hermetically package the sensor assembly, the sealed packaging box has a supporting structure and a boosting structure, the supporting structure is used for matching with the sensor assembly to limit a position of the sensor assembly in the sealed packaging box, and when the sensor assembly is moved away from the supporting structure under the action of the exertion device coupled to the sensor assembly, the boosting structure provides an acting force for moving away from the supporting structure to the exertion device; the sensor assembly is configured to be coupled to the electronic assembly to form an application assembly, the application assembly is configured to be provided on a target subject to perform analyte concentration monitoring on the target subject, the sensor assembly includes a sensor and a sharp object, the sensor is at least partially placed beneath the skin of the target subject and performs the analyte concentration monitoring on the target subject, and the sharp object is used for at least partially placing the sensor beneath the skin of the target subject; the electronic assembly is configured to be coupled to the sensor assembly to form the application assembly; and the exertion device is configured to be coupled to the sealed packaging box to couple the electronic assembly with the sensor assembly to form the application assembly, and exert the application assembly to the target subject. In this case, the sealed packaging box involved in the present disclosure may be utilized for encapsulating the sensor assembly of the analyte monitoring system, thereby meeting medical and health needs, and also improving the ease of use of the sealed packaging box in the analyte monitoring system.

According to the present disclosure, the sealed packaging box and the analyte monitoring system including the sealed packaging box are provided, which can improve the ease of use of the sealed packaging box.

### BRIEF DESCRIPTION OF THE DRAWINGS

The present disclosure will now be explained in further detail by way of examples only with reference to the accompanying drawings.
FIG. 1 is a schematic diagram showing an application scene of a sealed packaging box involved in an example of the present disclosure.
FIG. 2 is a schematic diagram showing the sealed packaging box and an exertion device which are involved in an example of the present disclosure.
FIG. 3 is an exploded schematic diagram showing an application assembly involved in an example of the present disclosure.
FIG. 4 is an exploded schematic diagram showing the sealed packaging box used in an analyte monitoring system involved in an example of the present disclosure.
FIG. 5 is a schematic diagram showing an overall structure of the sealed packaging box involved in an example of the present disclosure.
FIG. 6 is a schematic diagram showing a local structure of the sealed packaging box involved in an example of the present disclosure.

### DETAILED DESCRIPTION

Hereinafter, the preferred embodiments of the present disclosure will be described in detail with reference to the accompanying drawings. In the following description, the same reference numerals are given to the same components, and redundant description will be omitted. In addition, the accompanying drawings are only schematic diagrams, and the proportions of the dimensions of the components relative to each other, or the shapes of the components, and the like may be different from those in practice.

It should be noted that the terms "including" and "having", and any variations thereof, for example, a process, method, system, product, or equipment which includes or has a list of steps or units in the present disclosure, are not necessarily limited to those steps or units expressly listed, but may include or have other steps or units not expressly listed or inherent to such process, method, product, or equipment.

It should be noted that in this document, relative positional and relative directional terms, such as "upper", "upward", "lower", "downward", "up and down", "left side", "leftward", "left", "leftwards", "right side", "rightward", "right", "rightwards", "left and right", "front", "forward", "rear", "rearward", and "front and rear", refer to a normal operating posture and should not be regarded as restrictive.

The present disclosure relates to a sealed packaging box. In some examples, sometimes the sealed packaging box may also be referred to as a packaging box, a packaging shell, a sealed package, or a package. The present disclosure also relates to an analyte monitoring system. The analyte monitoring system may include the sealed packaging box involved in the present disclosure.

Hereinafter, the sealed packaging box and the analyte monitoring system including the sealed packaging box which are involved in the present disclosure will be described in detail with reference to the accompanying drawings.

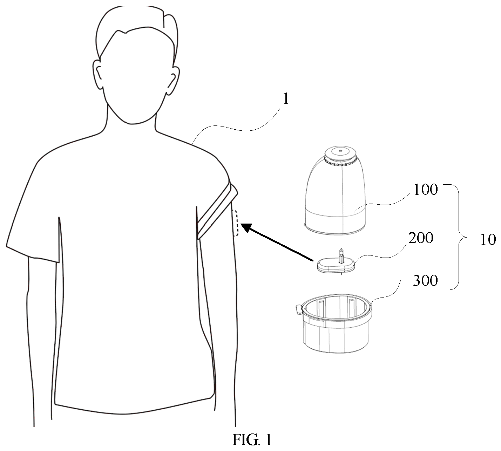

It should be noted that in order to more clearly illustrate a structure of the sealed packaging box, some unnecessary components have been omitted in FIG. 1, FIG. 2, FIG. 3, FIG. 4, FIG. 5 and FIG. 6, but this should not be construed as any feature on the present disclosure.

FIG. 1 is a schematic diagram showing an application scene of a sealed packaging box 300 involved in an example of the present disclosure.

Referring to FIG. 1, in some examples, an analyte monitoring system 10 may be used for performing analyte concentration monitoring on a target subject 1.

In some examples, an analyte may include at least one of DNA, RNA, acetylcholine, amylase, bilirubin, peroxide, cholesterol, prostate-specific antigens, chorionic gonadotropin, creatine kinase, thyroid stimulating hormone, growth hormone, lactate, creatine, creatinine, fructosamine, glucose, glutamine, ketone bodies, oxygen, prothrombin, and troponin. In some examples, when the analyte is glucose, the analyte monitoring system 10 may be a glucose monitoring system. In some examples, the analyte monitoring system 10 may be a continuous glucose monitoring system. The continuous glucose monitoring system may continuously monitor a glucose concentration of the target subject 1 in a period of time.

In some examples, the analyte monitoring system 10 may include an exertion device 100, an application assembly 200, and the sealed packaging box 300. The exertion device 100 may be configured to exert the application assembly 200 to the target subject 1. In some examples, the application assembly 200 may include a sensor assembly 210 and an electronic assembly 220. In some examples, the sensor assembly 210 may be at least partially placed beneath the skin of the target subject 1 and perform analyte concentration monitoring on the target subject 1.

In some examples, since the sensor assembly 210 needs to be at least partially placed beneath the skin of the target subject 1, in order to meet medical and health needs, the sensor assembly 210 needs to be hermetically packaged. In some examples, the sealed packaging box 300 involved in the present disclosure may be a sealed packaging box 300 used for encapsulating the sensor assembly 210 of the analyte monitoring system 10. The sealed packaging box 300 may be configured to accommodate and hermetically package the sensor assembly 210. In this case, the sealed packaging box 300 involved in the present disclosure may be utilized for encapsulating the sensor assembly 210 of the analyte monitoring system 10, thereby meeting the medical and health needs.

However, the application scene of the present disclosure is not limited thereto. In some examples, the sealed packaging box 300 may be applied in any scene where sealed packaging of articles is required. In some examples, the article may be a medical instrument including, but not limited to, a syringe, a needle, a mask, a surgical instrument, a stomatological instrument, an ophthalmic instrument, and the like. In some examples, the article may be a pharmaceutical, including but not limited to, antibiotics, biological products, and the like. In some examples, the articles may include food products, household products, laboratory products, cosmetics, and the like.

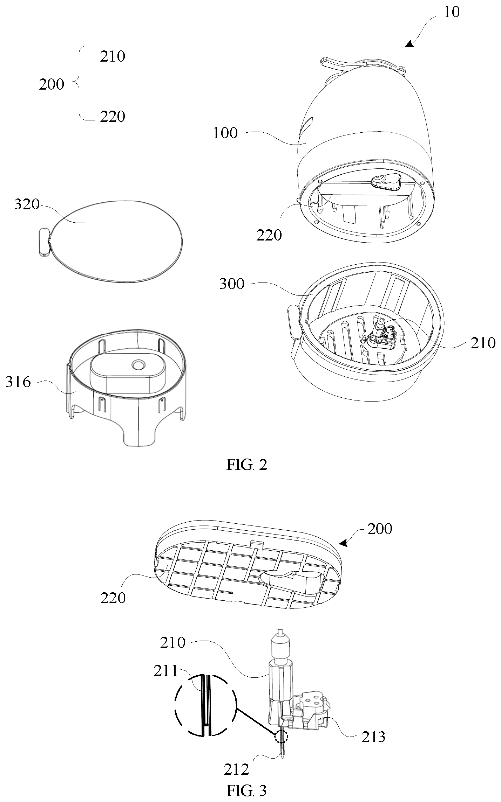

FIG. 2 is a schematic diagram showing the sealed packaging box 300 and an exertion device 100 which are involved in an example of the present disclosure.

Referring to FIG. 2, in some examples, the analyte monitoring system 10 may include the exertion device 100, an application assembly 200, and the sealed packaging box 300.

In some examples, the sensor assembly 210 may be accommodated in the sealed packaging box 300. The electronic assembly 220 may be accommodated in the exertion device 100. In some examples, the sensor assembly 210 may be configured to be coupled to the electronic assembly 220 to form the application assembly 200. In some examples, the electronic assembly 220 may be configured to be coupled to the sensor assembly 210 to form the application assembly 200. That is, the application assembly 200 may include the sensor assembly 210 and the electronic assembly 220. In some examples, the application assembly 200 may be configured to be provided on the target subject 1 to perform analyte concentration monitoring on the target subject 1.

Referring to FIG. 2, in some examples, a sealing portion 320 (described later) and a limiting cover 316 (described later) of the sealed packaging box 300 may be firstly removed in preparation for coupling the electronic assembly 220 in the exertion device 100 with the sensor assembly 210 in the sealed packaging box 300. In some examples, the exertion device 100 may be configured to be coupled to the sealed packaging box 300 to couple the electronic assembly 220 with the sensor assembly 210 to form the application assembly 200, and exert the application assembly 200 to the target subject 1. As a result, an analyte concentration of the target subject 1 may be monitored by the application assembly 200.

FIG. 3 is an exploded schematic diagram showing an application assembly 200 involved in an example of the present disclosure.

Referring to FIG. 3, in some examples, the application assembly 200 may include a sensor assembly 210 and an electronic assembly 220. The sensor assembly 210 may be at least partially placed beneath the skin of the target subject 1.

Referring to FIG. 3, in some examples, the sensor assembly 210 may include a sensor 211 and a sharp object 212. In some examples, the sensor 211 may be at least partially placed beneath the skin of the target subject 1 and perform analyte concentration monitoring on the target subject 1.

In some examples, the sharp object 212 may be used for at least partially placing the sensor 211 beneath the skin of the target subject 1. In some examples, the sharp object 212 may accommodate the sensor 211. The sharp object 212 may pierce the skin of the target subject 1, so that the sensor 211 is at least partially placed beneath the skin of the target subject 1. In this case, the sensor 211 may be guided to penetrate beneath the skin of the target subject 1 together by the sharp object 212.

In some examples, the sensor 211 and the sharp object 212 may be provided on a connecting base 213. In this case, the coupling of the sensor assembly 210 and the electronic assembly 220 through the connecting base 213 may be facilitated.

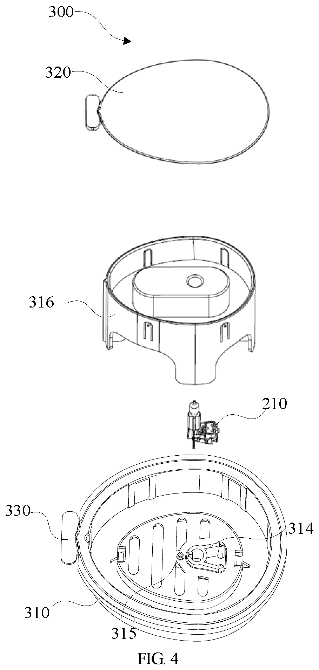

FIG. 4 is an exploded schematic diagram showing the sealed packaging box 300 used in an analyte monitoring system 10 involved in an example of the present disclosure.

In some examples, the sealed packaging box 300 may be configured to accommodate and hermetically package the sensor assembly 210. In this case, the sealed packaging box 300 involved in the present disclosure may be utilized for encapsulating the sensor assembly 210 of the analyte monitoring system 10, thereby meeting medical and health needs, and also improving the ease of use of the sealed packaging box 300 in the analyte monitoring system 10.

Referring to FIG. 4, in some examples, the sealed packaging box 300 may have a supporting structure 314 and a boosting structure 315. In some examples, the supporting structure 314 may be used for matching with the sensor assembly 210 to limit a position of the sensor assembly 210 in the sealed packaging box 300. In some examples, the sealed packaging box 300 may include a main body portion 310, a sealing portion 320, and a holding portion 330. In some examples, the main body portion 310 may have the supporting structure 314 and the boosting structure 315.

In some examples, the supporting structure 314 may releasably support the sensor assembly 210. In some examples, the supporting structure 314 may be tightly joined with the sensor assembly 210. In this case, undesired movement of the sensor assembly 210 may be limited by the supporting structure 314, thereby facilitating the alignment of the two when the sensor assembly 210 is subsequently picked up by using the exertion device 100. In some examples, the sensor assembly 210 tightly joined to the supporting structure 314 may disengage the retention of the supporting structure 314 under the action of an external force to move away from the supporting structure 314.

In some examples, the boosting structure 315 may be used for providing an acting force for moving away from the supporting structure 314 to the exertion device 100. When the sensor assembly 210 is moved away from the supporting structure 314 under the action of the exertion device 100 coupled to the sensor assembly 210, the boosting structure 315 may provide the acting force for moving away from the supporting structure 314 to the exertion device 100. In this case, it may be conducive to the successful disengagement of the sensor assembly 210 tightly joined with the supporting structure 314 from the supporting structure 314 under the action of the exertion device 100.

In some examples, the boosting structure 315 may include an elastic element. In some examples, the elastic element may be a spring or a leaf spring. The elastic element may be in a compressed state when the sensor assembly 210 is picked up by the exertion device 100, and an end of the elastic element may abut a surface of the exertion device 100 to provide an acting force for moving away from the supporting structure 314 to the exertion device 100 when the exertion device 100 carries the sensor assembly 210 away from the supporting structure 314.

In some examples, the sealed packaging box 300 may include a limiting cover 316. The limiting cover 316 may be used for limiting the position of the sensor assembly 210. The limiting cover 316 may be accommodated in a chamber 312 of the main body portion 310. In some examples, the limiting cover 316 may be housed above the sensor assembly 210. After the limiting cover 316 is moved away from the chamber 312, the sensor assembly 210 may be in a pickable state.

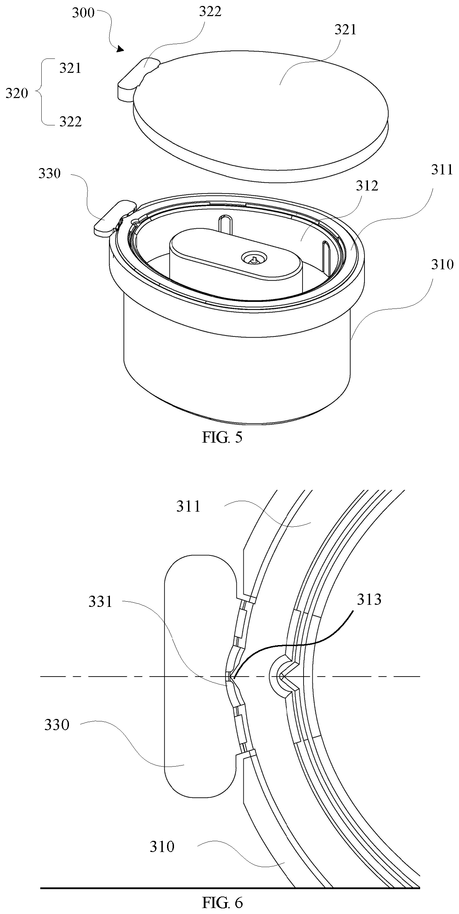

FIG. 5 is a schematic diagram showing an overall structure of the sealed packaging box 300 involved in an example of the present disclosure.

Referring to FIG. 5, in some examples, the sealed packaging box 300 may include a main body portion 310, a sealing portion 320, and a holding portion 330. The main body portion 310 may be used for accommodating articles (such as the sensor assembly 210). The sealing portion 320 may be used for covering the main body portion 310 of the sealed packaging box 300. The holding portion 330 may be a part which may be easily pinched or held. The holding portion 330 may be used for separating the sealing portion 320 from the opening portion 311.

In some examples, the main body portion 310 may have an opening portion 311 and a chamber 312 communicated with the outside via the opening portion 311. In some examples, the chamber 312 may be used for accommodating articles. The articles may be placed inside the chamber 312 from the outside via the opening portion 311. In some examples, the article may be the sensor assembly 210.

In some examples, the sealing portion 320 may be adhered to the opening portion 311 and seal the chamber 312. In some examples, the sealing portion 320 may be a relatively soft sealing film. In some examples, the sealing portion 320 may be a relatively hard sealing cover.

As described above, the holding portion 330 may be used for separating the sealing portion 320 from the main body portion 310. In some examples, the holding portion 330 may be used for separating the sealing portion 320 from the opening portion 311 of the main body portion 310.

In some examples, the holding portion 330 may form an integral structure with the sealing portion 320. In some examples, the holding portion 330 may be a part of the sealing portion 320. For example, the holding portion 330 may be a part of the sealing portion 320 extending away from the opening portion 311.

In some examples, the holding portion 330 may be at least partially attached to the sealing portion 320. In some examples, the holding portion 330 may be at least partially attached to a part of the sealing portion 320. For example, the holding portion 330 may be at least partially adhered to the sealing portion 320. In some examples, the holding portion 330 may be at least partially bonded to the sealing portion 320.

In some examples, the holding portion 330 may be at least partially attached to a peripheral edge of the sealing portion 320. For example, the holding portion 330 may be at least partially adhered to an edge of a periphery of the sealing portion 320. In some examples, the holding portion 330 may be at least partially bonded to the edge of the periphery of the sealing portion 320. In this case, the holding portion 330 may form an integral structure with the sealing portion 320, and peripheral edges of the holding portion 330 and the sealing portion 320 may be pinched simultaneously, so that the possibility that the sealing portion 320 slips off a hand of a user may be reduced, thereby being convenient for the user to utilize the holding portion 330 attached to the sealing portion 320 for driving the sealing portion 320 to move, so as to separate the sealing portion 320 from the opening portion 311, and then the ease of use of the sealed packaging box 300 may be improved. In addition, the sealing portion 320 is separated from the opening portion 311 from the peripheral edge of the sealing portion 320, so that when starting to separate the sealing portion 320 from the opening portion 311, it is convenient for the user to separate the sealing portion 320 from the opening portion 311 with a smaller force.

In some examples, the holding portion 330 may be easily pinched. In some examples, the sealing portion 320 may be separated from the opening portion 311 by utilizing the holding portion 330. For example, the user may pinch the holding portion 330 attached to the sealing portion 320 and move the holding portion 330 towards a direction away from the opening portion 311. Since the holding portion 330 is attached to the sealing portion 320, the user may utilize the holding portion 330 for driving the sealing portion 320 to move, so as to separate the sealing portion 320 from the opening portion 311. In this case, the holding portion 330 itself is easily pinched, so that the possibility that the sealing portion 320 slips off the hand of the user may be reduced.

In some examples, a hardness of the holding portion 330 may be not less than a hardness of the sealing portion 320. In some examples, a thickness of the holding portion 330 may be not less than a thickness of the sealing portion 320. In this case, the holding portion 330 may be easily pinched, and it may be convenient for the user to subsequently utilize the holding portion 330 attached to the sealing portion 320 for separating the sealing portion 320 from the opening portion 311, so that the ease of use of the sealed packaging box 300 may be improved.

In some examples, the hardness of the holding portion 330 may be not less than the hardness of the sealing portion 320, and the thickness of the holding portion 330 may be not less than the thickness of the sealing portion 320. In this case, the holding portion 330 may be more easily pinched.

In some examples, anti-slip texture may be provided on a surface of the holding portion 330. In this case, a friction coefficient of the holding portion 330 may be increased, and the possibility that the holding portion 330 slips off the hand of the user may be reduced.

In some examples, anti-slip texture may be provided on a surface of the holding portion 330 facing away from the sealing portion 320. In some examples, the anti-slip texture may include at least one of chevron, wavy, reticulated, granulate, and linear texture. In this case, the anti-slip texture is provided on the surface of the holding portion 330 facing away from the sealing portion 320, so that the tightness of adhesion between the sealing portion 320 and the holding portion 330 may be improved, and the sealability of the sealed packaging box 300 may be improved as compared to the manner in which the anti-slip texture is provided on a surface of the holding portion 330 attached to the sealing portion 320.

In some examples, a shape of the holding portion 330 may be a shape which is easily pinched or held. For example, the shape of the holding portion 330 may be annular, circular, oval, or rectangular.

In some examples, the sealing portion 320 may have a part matching with the holding portion 330. For example, the sealing portion 320 may have a part which is specifically adhered to the holding portion 330.

With continued reference to FIG. 5, in some examples, the sealing portion 320 may have a covering portion 321 corresponding to the opening portion 311, and a tearing portion 322. The tearing portion 322 may extend outwards from the covering portion 321 and beyond the peripheral edge of the opening portion 311. In this case, the tearing portion 322 extends outwards from the covering portion 321 and beyond the peripheral edge of the opening portion 311, so that a degree of coverage of the opening portion 311 by the sealing portion 320 may be increased, and thus the sealability of the sealed packaging box 300 may be improved.

In some examples, when the sealing portion 320 has the tearing portion 322, the holding portion 330 may be at least partially attached to the tearing portion 322. In this case, the holding portion 330 may be utilized for driving the tearing portion 322 to move towards a direction away from the opening portion 311, so as to separate the sealing portion 320 from the opening portion 311.

In some examples, the holding portion 330 may be at least partially adhered to the tearing portion 322. In some examples, the holding portion 330 may be at least partially bonded to the tearing portion 322.

In some examples, the sealing portion 320 may be hot-pressed to the opening portion 311 and the holding portion 330. In this case, the tightness of adhesion between the sealing portion 320 and the opening portion 311 and between the sealing portion 320 and the holding portion 330 may be improved, and the sealability of the sealed packaging box 300 may be improved.

In some examples, the sealing portion 320 may be bonded to the holding portion 330. In some examples, the sealing portion 320 may be glued to the holding portion 330. That is, there may be a gluing layer between the sealing portion 320 and the holding portion 330.

In some examples, the holding portion 330 may be formed integrally with the opening portion 311. In this case, machining may be facilitated, and the sealability of the sealed packaging box 300 may be improved.

In some examples, the holding portion 330 may be easily separated from the opening portion 311. For example, the connection between the holding portion 330 and the opening portion 311 may be easily broken.

In some examples, a breaking stress between the holding portion 330 and the opening portion 311 may be less than a breaking stress of the opening portion 311. In some examples, the breaking stress between the holding portion 330 and the opening portion 311 may be less than a breaking stress of the holding portion 330. In this case, it may be conducive to breaking the connection between the holding portion 330 and the opening portion 311 by the user, and thus be convenient for the user to subsequently pinch the holding portion 330 attached to the sealing portion 320 and move the holding portion 330 towards a direction away from the opening portion 311, so as to separate the sealing portion 320 from the opening portion 311.

FIG. 6 is a schematic diagram showing a local structure of the sealed packaging box 300 involved in an example of the present disclosure.

Referring to FIG. 6, in some examples, the holding portion 330 may have a connecting portion 331 connected to the opening portion 311. In some examples, a thickness of the connecting portion 331 may be less than a thickness of the opening portion 311. In this case, a breaking stress between the holding portion 330 and the opening portion 311 may be reduced, thereby being conducive to breaking the holding portion 330 and the opening portion 311 from a position of the connecting portion 331 by the user with a smaller force, and thus being convenient for the user to subsequently utilize the holding portion 330 for separating the sealing portion 320 from the opening portion 311.

In some examples, the connecting portion 331 may be formed by weakening a part of a material of the holding portion 330. For example, the connecting portion 331 may have a recess. The recess may not pass through an upper surface and a lower surface of the holding portion 330. In some examples, the connecting portion 331 may have an indentation. The indentation may not pass through the upper surface and the lower surface of the holding portion 330. In this case, the breaking stress of the connecting portion 331 may be reduced, thereby being conducive to breaking the connection between the holding portion 330 and the opening portion 311 by the user with a smaller force, and also reducing the possibility that the holding portion 330 is accidentally broken.

In some examples, the connecting portion 331 may have a through line. The through line may pass through the upper surface and the lower surface of the holding portion 330. In this case, the breaking stress of the connecting portion 331 may be further reduced, and the connecting strength between the holding portion 330 and the opening portion 311 may be reduced, thereby being conducive to breaking the connection between the holding portion 330 and the opening portion 311 by the user with a smaller force.

In some examples, the through line may be a dashed line. In some examples, the through line may be a straight line. In some examples, the through line may be a curve. In some examples, there may be a plurality of through lines, for example, 1, 2, 3, 4, 5, 6, or 7 through lines.

In some examples, the connecting portion 331 may have a through hole. The through hole may pass through the upper surface and the lower surface of the holding portion 330. In some examples, the through hole may be a substantially circular hole. In this case, a broken edge of the circular hole is relatively smooth, thereby being conducive to reducing the possibility that the user is injured by cutting after the holding portion 330 is broken.

In some examples, the main body portion 310 may have a part matching with the connecting portion 331.

In some examples, the main body portion 310 may have a protrusion 313. The protrusion 313 may be formed by protruding from the opening portion 311 towards the holding portion 330. A peripheral curvature of the protrusion 313 may be greater than a peripheral curvature of the opening portion 311. The peripheral curvature may refer to a curvature of a peripheral part of the protrusion 313 or the opening portion 311 away from the chamber 312. In this case, as compared to a case where there is no protrusion 313, when starting to separate the sealing portion 320 from the opening portion 311, areas of the sealing portion 320 and the opening portion 311 needing to be separated may be reduced, thereby being convenient for the user to separate the sealing portion 320 from the opening portion 311 with a smaller force.

In some examples, when the main body portion 310 has the protrusion 313, the connecting portion 331 may be a connecting point connected to the protrusion 313 of the main body portion 310. In this case, a breaking stress of the connecting portion 331 may be decreased by reducing a volume of the connecting portion 331, and the holding portion 330 may be easily broken from the connecting portion 331.

Preferred examples of the present disclosure, including the known optimal structures used for implementing the present disclosure, will be described below.

Referring back to FIG. 5, in some examples, the covering portion 321 may be formed integrally with the tearing portion 322. The holding portion 330 may be formed integrally with the opening portion 311. In some examples, the covering portion 321 may be hot-pressed to the opening portion 311, and the tearing portion 322 may be hot-pressed to the holding portion 330. In this case, the covering portion 321 is formed integrally with the tearing portion 322, and the holding portion 330 is formed integrally with the opening portion 311, so that machining may be facilitated, and the sealability of the sealed packaging box 300 may be improved. In addition, the covering portion 321 is hot-pressed to the opening portion 311, and the tearing portion 322 is hot-pressed to the holding portion 330, so that the tightness of adhesion between the sealing portion 320 and the opening portion 311 and between the sealing portion 320 and the holding portion 330 may be improved, and the sealability of the sealed packaging box 300 may be improved.

Referring to FIG. 6, in some examples, the main body portion 310 may have a protrusion 313 formed by protruding from the opening portion 311 towards the holding portion 330, and a peripheral curvature of the protrusion 313 may be greater than a peripheral curvature of the opening portion 311. The holding portion 330 may have a connecting point connected to the protrusion 313. In this case, as compared to a case where there is no protrusion 313, when starting to separate the sealing portion 320 from the opening portion 311, areas of the sealing portion 320 and the opening portion 311 needing to be separated may be reduced, thereby being convenient for the user to separate the sealing portion 320 from the opening portion 311 with a smaller force. In addition, the holding portion 330 has the connecting point connected to the protrusion 313, and the connecting point is a connecting portion 331, so that a breaking stress of the connecting portion 331 may be decreased by reducing a volume of the connecting portion 331, and the holding portion 330 may be easily broken from the connecting portion 331.

In some examples, a thickness of the connecting point may be less than a thickness of the opening portion 311. In this case, the breaking stress between the holding portion 330 and the opening portion 311 may be reduced, thereby being conducive to breaking the holding portion 330 and the opening portion 311 from a position of the connecting portion 331 by the user.

In some examples, a hardness of the holding portion 330 may be greater than a hardness of the sealing portion 320. In this case, the holding portion 330 may be easily pinched.

In some examples, anti-slip texture may be provided on a surface of the holding portion 330 facing away from the sealing portion 320. In this case, a friction coefficient of the holding portion 330 may be increased, and the possibility that the holding portion 330 slips off the hand of the user may be reduced, so that the tightness of adhesion between the sealing portion 320 and the holding portion 330 may also be improved, and thus the sealability of the sealed packaging box 300 may be improved as compared to the manner in which the anti-slip texture is provided on a surface of the holding portion 330 attached to the sealing portion 320.

The present disclosure relates to a sealed packaging box 300 and an analyte monitoring system 10 including the sealed packaging box 300. The sealed packaging box 300 may include a main body portion 310, a sealing portion 320, and a holding portion 330, wherein the main body portion 310 has an opening portion 311 and a chamber 312 communicated with the outside via the opening portion 311, the sealing portion 320 is adhered to the opening portion 311 and seals the chamber 312, and the holding portion 330 is at least partially attached to a peripheral edge of the sealing portion 320 to separate the sealing portion 320 from the opening portion 311 by utilizing the holding portion 330.

In the sealed packaging box 300 involved in the present disclosure, the holding portion 330 is at least partially attached to a peripheral edge of the sealing portion 320. In this case, the holding portion 330 and the sealing portion 320 may be pinched simultaneously, so that the possibility that the sealing portion 320 slips off a hand of a user may be reduced, thereby being convenient for the user to utilize the holding portion 330 attached to the sealing portion 320 for driving the sealing portion 320 to move, so as to separate the sealing portion 320 from the opening portion 311, and then the ease of use of the sealed packaging box 300 may be improved.

Although the present disclosure has been described in detail with reference to the accompanying drawings and examples, it should be understood that the above description does not limit the present disclosure in any way. A person skilled in the art would have been able to make modifications and variations to the present disclosure as required without departing from the true spirit and scope of the present disclosure, and these modifications and variations all fall within the scope of the present disclosure.

## Claims

1. A sealed packaging box, comprising a main body portion, a sealing portion, and a holding portion, wherein the main body portion has an opening portion and a chamber communicated with the outside via the opening portion, the sealing portion is adhered to the opening portion and seals the chamber, and the holding portion is at least partially attached to a peripheral edge of the sealing portion to separate the sealing portion from the opening portion by utilizing the holding portion.

2. The sealed packaging box according to claim 1, wherein,
the sealing portion has a covering portion corresponding to the opening portion and a tearing portion extending outwards from the covering portion and beyond a peripheral edge of the opening portion, and the holding portion is at least partially attached to the tearing portion.

3. The sealed packaging box according to claim 1, wherein,
the holding portion is formed integrally with the opening portion, and a breaking stress between the holding portion and the opening portion is smaller than a breaking stress of the opening portion or a breaking stress of the holding portion.

4. The sealed packaging box according to claim 3, wherein,
the holding portion has a connecting portion connected to the opening portion, and a thickness of the connecting portion is less than a thickness of the opening portion.

5. The sealed packaging box according to claim 1, wherein,
anti-slip texture is provided on a surface of the holding portion facing away from the sealing portion.

6. The sealed packaging box according to claim 1, wherein,
a hardness of the holding portion is not less than a hardness of the sealing portion and/or a thickness of the holding portion is not less than a thickness of the sealing portion.

7. The sealed packaging box according to claim 1, wherein,
the sealing portion is hot-pressed to the opening portion and the holding portion.

8. The sealed packaging box according to claim 1, wherein,
the main body portion has a protrusion formed by protruding from the opening portion towards the holding portion, and a peripheral curvature of the protrusion is greater than a peripheral curvature of the opening portion.

9. The sealed packaging box according to claim 1, wherein,
the sealing portion has a covering portion corresponding to the opening portion and a tearing portion extending outwards from the covering portion and beyond an edge of the opening portion, the covering portion is formed integrally with the tearing portion, the covering portion is hot-pressed to the opening portion, and the tearing portion is hot-pressed to the holding portion,
the holding portion is formed integrally with the opening portion, the main body portion has a protrusion formed by protruding from the opening portion towards the holding portion, and a peripheral curvature of the protrusion is greater than a peripheral curvature of the opening portion, and
the holding portion has a connecting point connected to the protrusion, a thickness of the connecting point is less than a thickness of the opening portion, a hardness of the holding portion is greater than a hardness of the sealing portion, and anti-slip texture is provided on a surface of the holding portion facing away from the sealing portion.

10. An analyte monitoring system comprising the sealed packaging box according to any one of claims 1 to 9, a sensor assembly, an electronic assembly, and an exertion device, wherein,
the sealed packaging box is configured to accommodate and hermetically package the sensor assembly, the sealed packaging box has a supporting structure and a boosting structure, the supporting structure is used for matching with the sensor assembly to limit a position of the sensor assembly in the sealed packaging box, and when the sensor assembly is moved away from the supporting structure under the action of the exertion device coupled to the sensor assembly, the boosting structure provides an acting force for moving away from the supporting structure to the exertion device;
the sensor assembly is configured to be coupled to the electronic assembly to form an application assembly, the application assembly is configured to be provided on a target subject to perform analyte concentration monitoring on the target subject, the sensor assembly comprises a sensor and a sharp object, the sensor is at least partially placed beneath skin of the target subject and performs the analyte concentration monitoring on the target subject, and the sharp object is used for at least partially placing the sensor beneath the skin of the target subject;
the electronic assembly is configured to be coupled to the sensor assembly to form the application assembly; and
the exertion device is configured to be coupled to the sealed packaging box to couple the electronic assembly with the sensor assembly to form the application assembly, and exert the application assembly to the target subject.
